(19) Europäisches Patentamt
European Patent Office
Office européen des brevets

(11) Veröffentlichungsnummer: **0 039 864**
**A1**

(12) # EUROPÄISCHE PATENTANMELDUNG

(21) Anmeldenummer: **81103339.8**

(22) Anmeldetag: **04.05.81**

(51) Int. Cl.³: **C 07 D 251/34**
C 09 D 3/72, C 08 G 18/75

(30) Priorität: **13.05.80 DE 3018198**

(43) Veröffentlichungstag der Anmeldung:
**18.11.81 Patentblatt 81/46**

(84) Benannte Vertragsstaaten:
**BE DE FR GB IT NL**

(71) Anmelder: **BAYER AG**
**Zentralbereich Patente, Marken und Lizenzen**
**D-5090 Leverkusen 1, Bayerwerk(DE)**

(72) Erfinder: **Breidenbach, Peter, Dr.**
**Maarweg 33**
**D-5000 Köln 41 (Braunsfeld)(DE)**

(72) Erfinder: **Bock, Manfred, Dr.**
**Haydnstrasse 18**
**D-5090 Leverkusen(DE)**

(72) Erfinder: **Pedain, Josef, Dr.**
**Haferkamp 6**
**D-5000 Köln 80(DE)**

(54) **Neue Isocyanato-isocyanurate, ein Verfahren zu ihrer Herstellung, sowie ihre Verwendung als Isocyanat-Komponente in Polyurethan-Lacken.**

(57) Die Erfindung betrifft neue Isocyanato-isocyanurate, ein Verfahren zu ihrer Herstellung, bei welchem man einen Teil der Isocyanatgruppen von 3 (4), 8 (9)-Bis(-isocyanatomethyl)-tricyclo[5,2,1,$0^{2,6}$]-decan in Gegenwart von die Trimerisierung von Isocyanatgruppen beschleunigenden Katalysatoren trimerisiert und die Verwendung der neuen Verbindung, gegebenenfalls in mit Blockierungsmitteln für Isocyanatgruppen blockierter Form, als Isocyanatkomponente in Polyurethanlacken.

**EP 0 039 864 A1**

0039864

BAYER AKTIENGESELLSCHAFT 5090 Leverkusen, Bayerwerk
Zentralbereich Patente, Marken und Lizenzen Wr/mo-C

Neue Isocyanato-isocyanurate, ein Verfahren zu ihrer Herstellung, sowie ihre Verwendung als Isocyanat-Komponente in Polyurethan-Lacken

Die vorliegende Erfindung betrifft neue Isocyanato-isocyanurate auf Basis von Bis-(isocyanatomethyl)-tricyclo/$\bar{5},2,1,0^{2,6}$/-decan, ein Verfahren zur Herstellung der neuen Verbindungen durch katalytische Trimerisierung der genannten Diisocyanate, sowie die Verwendung dieser neuen Verbindungen, gegebenenfalls in mit Blockierungsmitteln für Isocyanat-Gruppen blockierter Form, als Isocyanatkomponente in Polyurethan-Lacken.

Es ist bekannt, Isocyanursäurederivate durch katalytische Trimerisierung organische Isocyanate herzustellen. Durch Trimerisierung organischer Diisocyanate erhält man überwiegend Triisocyanate, wenn durch geeignete Maßnahmen, wie etwa Einsatz von Diisocyanaten mit Isocyanatgruppen unterschiedlicher Reaktivität und/oder vorzeitiger Abbruch der Reaktion, die Bildung höhermolekularer Produkte vermieden, bzw. unterdrückt wird. Derartige Polyisocyanate, ggf. durch bekannte Methoden wie etwa Dünnschichtdestillation weitgehend von Monomeren befreit, sind als lösliche, polyfunktionelle, auf Grund ihres hohen Dampfdrucks physiologisch unbedenklich anzuwendende Isocyanate mit hohem NCO-Gehalt

besonders als Isocyanatvernetzer für schnelltrocknende Zweikomponenten-Polyurethanlacke interessant.

Für lichtechte Lacke sind insbesondere Isocyanato-isocyanurate mit nicht-aromatisch, d.h. aliphatisch oder cycloaliphatisch gebundenen Isocyanatgruppen von Interesse, da insbesondere derartige Verbindungen die Herstellung von Beschichtungen einer hohen UV-Beständigkeit gestatten. Bislang sind insbesondere zwei Arten derartiger nicht-aromatischer Isocyanato-isocyanurate bekannt geworden, und zwar einerseits flüssige Weichharze mit reaktiven (primären) NCO-Gruppen, die für lösungsmittelarme bzw. -freie Beschichtungssysteme Verwendung finden und andererseits bei Raumtemperatur feste Isocyanato-isocyanurate, die wenig reaktive (sekundäre) NCO-Gruppen aufweisen. Beispiele für die erstgenannte Art sind insbesondere die Trimerisate des Hexamethylendiisocyanates, die u.a. in GB-PS 809 809, den DE-OSen 2 616 415 oder 2 325 826 oder in den DE-ASen 1 150 080 und 2 226 191 beschrieben sind. Diese Verbindungen sind wegen ihres niedrigen Schmelzpunktes für Pulverlacke ungeeignet. Außerdem können sie wegen ihrer geringen Verträglichkeit mit unpolaren Lösungsmitteln nur bedingt für lufttrocknende, in Benzin lösliche Autoreparaturlacke eingesetzt werden. Zur zweiten, oben genannten Art gehören insbesondere die Trimerisate des 1-Isocyanato-3,3,5-trimethyl-5-isocyanatomethyl-cyclohexans (Isophoron-Diisocyanat), deren Herstellung beispielsweise in den GB-PSen 1 391 066 oder 1 386 399 oder in der DE-OS 2 325 826 beschrieben ist oder die Trimerisate des 4,4'-Diisocyanato-dicyclohexyl-methans,

deren Herstellung u.a. in der DE-OS 2 644 684 erwähnt wird. Solche Trimerisate sind gut für Pulverlacke und, bei guter Lösungsmittelverträglichkeit und guter physikalischer Antrocknung, für Autoreparaturlacke geeignet. Ein Nachteil dieser Isocyanato-isocyanurate ist jedoch in ihrer Reaktionsträgheit zu sehen, die dazu führt, daß die Trocknungsgeschwindigkeit bei Raumtemperatur von Polyurethanlacken, die diese Polyisocyanate als wesentliche Isocyanatkomponente enthalten, den Forderungen der Praxis nicht vollauf genügt.

Es war die Aufgabe der vorliegenden Erfindung, neue Isocyanato-isocyanurate zur Verfügung zu stellen, die sowohl einen hohen Schmelzpunkt und eine gute Lösungsmittelverträglichkeit als auch eine für eine rasche chemische Trocknung bei Raumtemperatur ausreichende Reaktivität aufweisen.

Diese Aufgabe konnte durch die Bereitstellung der nachstehend näher beschriebenen erfindungsgemäßen Verbindungen bzw. durch die Bereitstellung des nachstehend näher beschriebenen Verfahrens zu ihrer Herstellung gelöst werden.

Gegenstand der vorliegenden Erfindung sind Isocyanato-isocyanurate der Formel

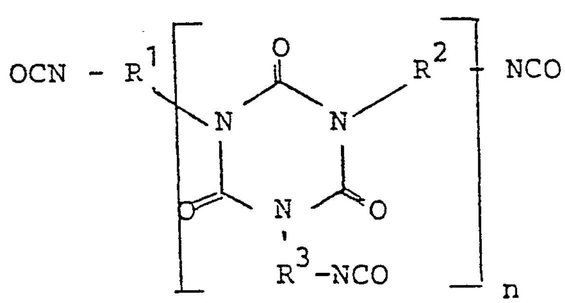

in welcher

$R^1$, $R^2$, und $R^3$ für gleich oder verschiedene Reste stehen und isomere Kohlenwasserstoffreste der Formeln

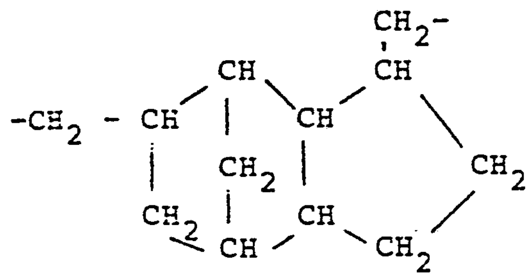

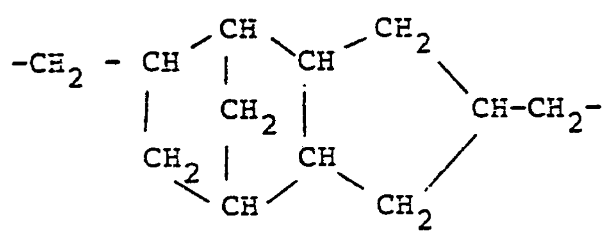

oder

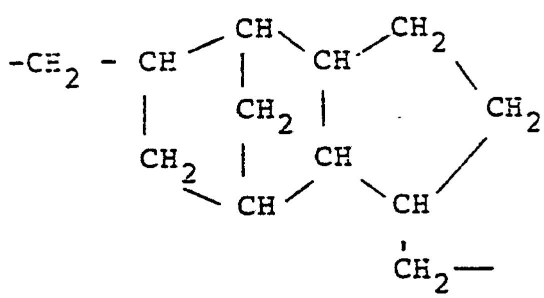

bedeuten

und

n für eine ganze oder (im statistischen Mittel) gebrochene Zahl von 1 bis 5 steht.

Gegenstand der vorliegenden Erfindung ist auch ein Verfahren zur Herstellung derartiger Isocyanato-isocyanurate, welches dadurch gekennzeichnet ist, daß man einen Teil der Isocyanatgruppen von 3 (4), 8, (9)-Bis(-isocyanatomethyl)-tricyclo /5,2,1,$0^{2,6}$7-decan (TCDI) in Gegenwart von die Trimerisierung von Isocyanatgruppen beschleunigenden Katalysatoren trimerisiert.

Gegenstand der vorliegenden Erfindung ist schließlich auch die Verwendung der neuen Isocyanato-isocyanurate, gegebenenfalls in mit Blockierungsmitteln für Isocyanatgruppen blockierter Form, als Isocyanatkomponente in Polyurethan-Lacken.

Ausgangsmaterialien für das erfindungsgemäße Verfahren sind Diisocyanate der Formel

$$OCN - R^1 - NCO$$

bzw. (bei unterschiedlicher Bedeutung der Reste $R^1$, $R^2$ und $R^3$) beliebige Gemische derartiger Diisocyanate mit Diisocyanaten der Formeln

$$OCN - R^2 - NCO$$

und/oder

$$OCN - R^3 - NCO$$

wobei
$R^1$, $R^2$ und $R^3$ die obengenannte Bedeutung haben.

Besonders gut als Ausgangsmaterial für das erfindungsgemäße Verfahren geeignet sind solche Isomerengemische, die beispielsweise gemäß Beispiel 2 der DE-OS 1 645 595 aus dimerem Cyclopentadien durch die Reaktionsfolge Hydroformylierung, reduktive Aminierung und Phosgenierung hergestellt werden können (siehe auch Chem. Zeitung 98, 70 (1975) und DE-OS 2 819 980). Ein so erhaltenes Isomerengemisch siedet bei 126 - 127°C (0,15 mm Hg) und ist nach der Destillation eine farblose Flüssigkeit.

Für das erfindungsgemäße Verfahren geeignete Katalysatoren sind insbesondere mindestens eine Hydroxyalkylgruppe aufweisende quaternäre Ammoniumhydroxide der Formel

$$R^5-\underset{R^4}{\overset{R^6}{N^{\oplus}}}-CHR^7-CHR^7-OH \quad OH^{\ominus}$$

in welcher

$R^4$, $R^5$ und $R^6$ für gleiche oder verschiedene Reste stehen und gegebenenfalls Hydroxyl-substituierte Alkylgruppen mit 1 bis 20 Kohlenstoffatomen, gegebenenfalls mit Hydroxylgruppen substituierte Cycloalkylreste mit 4 bis 15 Kohlenstoffatomen, gegebenenfalls mit Hydroxylgruppen substituierte Alkylreste mit 7 bis 15 Kohlenstoffatomen oder gegebenenfalls mit Hydroxylgruppen substituierte Arylreste mit 6 bis 15 Kohlenstoffatomen bedeuten, wobei zwei

der genannten Reste $R^4$, $R^5$ oder $R^6$ auch zusammen mit dem Stickstoffatom und gegebenenfalls zusammen mit einem Sauerstoff- oder einem weiteren Stickstoff-Heteroatom einen heterocyclischen Ring mit 4-6 Kohlenstoffatomen bilden können, oder wobei die Reste $R^4$, $R^5$ und $R^6$ jeweils für Ethylenreste stehen, die zusammen mit dem quartären Stickstoffatom ein bicyclisches Triethylen-diamin-Gerüst bilden,

$R^7$ für Wasserstoff und/oder eine Alkylgruppe mit 1 bis 12 Kohlenstoffatomen, eine Cycloalkylgruppe mit 5 bis 7 Kohlenstoffatomen, eine Aralkylgruppe mit 7 bis 10 Kohlenstoffatomen, eine Arylgruppe mit 6 bis 10 Kohlenstoffatomen, oder eine $R^8$-O-$(CH_2)_n$-Gruppe darstellt, in welcher $R^8$ für Wasserstoff, einen Alkylrest mit 1 bis 12 Kohlenstoffatomen, einen Cycloalkylrest mit 4 bis 10 Kohlenstoffatomen, einen Aralkylrest mit 7 bis 10 Kohlenstoffatomen oder einen Arylrest mit 6 bis 10 Kohlenstoffatomen steht und n eine ganze Zahl von 1 bis 6 bedeutet.

Zu den bevorzugten erfindungsgemäß einzusetzenden Katalysatoren gehören Verbindung der letztgenannten Formel, bei welchen

$R^4$, $R^5$ und $R^6$ für gleiche oder verschiedene Alkylreste mit 1 bis 4 Kohlenstoffatomen stehen

und

$R^7$ Wasserstoff bedeutet.

Ein besonders bevorzugter Katalysator ist N,N,N-Trimethyl-N-(2-hydroxyethyl)-ammonium-hydroxid.

Die für das erfindungsgemäße Verfahren als Katalysatoren geeigneten Verbindungen werden hergestellt aus tert. Aminen wie beispielsweise Trimethylamin, Tributylamin, 2-Dimethyl-aminoethanol, Triethanolamin, Dodecyldimethylamin, N,N-Dimethylcyclohexylamin, N-Methylpyrrolidin, N-Methylmorpholin oder 1,4-Diazabicyclo-2,2,2-octan und Alkylenoxiden wie Ethylenoxid, Propylenoxid, Butylenoxid-1,2, Styroloxid, Methoxy-, Ethoxy- oder Phenoxypropylenoxid.

Die Darstellung der Katalysatoren erfolgt in an sich bekannter Weise durch Umsetzung von Alkylenoxid und tert. Amin in wäßrig-alkoholischem Medium (vgl. US-PS 3 995 997, Kol. 2, Zeilen 19 - 44). Als Alkohole eignen sich beispielsweise Methanol, Ethanol, Propanol oder tert.-Butanol. Destillierbare Anteile, wie das Wasser oder das an der Umsetzung mit Alkylenoxid nicht teilnehmende tert.-Butanol, werden anschließend entfernt. Der Gehalt an quartärer Base wird auf analytischem Wege, beispielsweise titrimetrisch ermittelt, so daß die für die Trimerisierung jeweils benötigte Menge bestimmt werden kann.

Die Katalysatoren werden im allgemeinen in einer Menge von 0,01 bis 1 Gew.-%, bevorzugt von 0,03 bis 0,3 Gew.-% bezogen auf eingesetztes Diisocyanat angewendet. Sie können in reiner Form oder als Lösung eingesetzt werden. Als Lösungsmittel eignen sich je nach Art des Katalysators

beispielsweise Toluol, Dimethylformamid, Dimethylsulfoxid oder auch Mischungen dieser Lösungsmittel. Bei der Mitverwendung von Carbamidsäurederivate bildenden Hydroxyverbindungen als Co-Katalysatoren (s.u.) ist es von Vorteil, diese als Lösungsmittel der Katalysatoren zu verwenden. Als solche kommen beispielsweise in Frage Methanol, Ethanol, 2-Ethyl-hexanol oder Glykole wie Ethandiol, Butandiol oder 2-Ethyl-hexandiol.

Die Trimerisierung verläuft bei entsprechender Dosierung des Katalysators langsam und stetig (ohne Inkubationszeit) ab. Hierdurch kann lösungsmittelfrei gearbeitet werden. Eine nicht genügend fortgeschrittene Trimerisierung läßt sich nachkatalysieren bis der gewünschte Trimerisierungsgrad erreicht ist, was nach den Trimerisierungsverfahren des Standes der Technik nicht ohne weiteres möglich ist. Aufgrund der Thermoinstabilität der Katalysatoren ist eine Beendigung der Trimerisierung durch Abstopper nicht erforderlich. Störende Trübungen durch Salzbildung mit Abstoppern treten dementsprechend bei den Trimerisatprodukten auch in großer Verdünnung nicht auf. Ein weiterer Vorteil der Thermoinstabilität der erfindungsgemäß einzusetzenden Katalysatoren ist in dem Umstand zu sehen, daß ein unkontrolliertes Durchpolymerisieren des Reaktionsansatzes praktisch ausgeschlossen ist, da durch die in einem solchen Falle zunächst eintretende starke Temperaturerhöhung automatisch die Zersetzungstemperatur des eingesetzten Katalysators erreicht und damit ein Abbruch der Reaktion bewirkt würde.

Die Mitverwendung von Co-Katalysatoren ist beim erfindungsgemäßen Verfahren möglich, jedoch nicht erforderlich. Als Co-Katalysatoren kommen Substanzen in Frage von denen bekannt ist, daß sie Isocyanate polymerisieren. Sie werden in Mengen von 1-90, vorzugsweise 1-50 Gew.-% des verwendeten Katalysators eingesetzt. Als Co-Katalysatoren kommen z.B. folgende Substanzen in Frage: tert. Amine, wie Triethylamin, Tributylamin, N-Methylmorpholin, N-ethyl-morpholin, N-Cocomorpholin, N,N,N',N'-Tetramethylethylendiamin, 1,4-Diaza-bicyclo-(2,2,2)-octan, N-Methyl-N'-dimethyl-aminoethyl-piperazin, N,N-Dimethylbenzylamin, Bis-(N,N-diethylaminoethyl)-adipat, N,N-Diethylbenzylamin, Pentamethyldiethylentriamin, N,N-Dimethylcyclohexylamin, N,N,N',N'-Tetramethyl-1,3-butandiamin, N,N-Dimethyl-ß-phenylethylamin, 1,2-Dimethylimidazol, 2-Methylimidazol.

Als Co-Katalysatoren eignen sich ebenfalls Mannichbasen aus sekundären Aminen, wie Dimethylamin, Diethylamin oder Morpholin und Aldehyden, vorzugsweise Formaldehyd, oder Ketonen wie Aceton, Methylethylketon oder Cyclohexanon und Phenolen, wie Phenol, Nonylphenol oder Bisphenol. Als Zusatzkatalysatoren eignen sich auch einige gegenüber Isocyanatgruppen aktive Wasserstoffatome aufweisende tert. Amine, wie beispielsweise Triethanolamin, Triisopropanolamin, N-Methyldiethanolamin, N,N-Dimethyl-ethanolamin, sowie deren Umsetzungsprodukte mit Alkylenoxiden, wie Propylenoxid und/oder Ethylenoxid. Als Co-Katalysatoren kommen ferner aliphatische, araliphatische und gemischt aliphatisch-aromatische Phosphine in Frage, z.B. Triethylphosphin, Tri-n-butylphospin,

Dimethylbenzylphosphin, Dimethylphenylphosphin, Tribenzylphosphin oder P-Butyl-phosphacyclopentan.

Weiter eignen sich Silaamine mit Kohlenstoff-Silicium-Bindungen, wie sie z.B. in der deutschen Patentschrift 1 229 290 beschrieben sind, z.B. 2,2,4-Trimethyl-2-silamorpholin oder 1,3-Diethylaminomethyl-tetramethyl-disiloxan.

Die Mitverwendung von Carbamidsäureestern analog GB-PS 949 253 oder DE-AS 1 013 869 ist ebenfalls möglich. Die mitzuverwendenden Carbamidsäureester werden durch Umsetzung von aliphatischen, araliphatischen oder aromatischen Mono- oder Polyhydroxylverbindungen mit Mono- oder Polyisocyanaten, zweckmäßigerweise mit dem hier verwendeten TCDI hergestellt. Es ist ohne Einfluß auf den Polymerisationsverlauf, ob ein vorgebildeter und isolierter Carbamidsäureester dem zu polymerisierenden TCDI zugesetzt wird oder ob dieser erst während der Trimerisierungsreaktion gebildet wird, wenn man beispielsweise die Hydroxyverbindung zusammen mit dem Katalysator, etwa als Lösung, in das Isocyanat einbringt. Geeignete Hydroxyverbindungen, die mit TCDI zu den als Co-Katalysatoren wirksamen Carbamidsäurederivaten reagieren und gleichzeitig Löser der eigentlich als Trimierisierungskatalysator wirksamen quartären Stickstoffbasen darstellen, sind beispielsweise Methanol, Ethanol, 2-Ethylhexanol oder Glykole, wie Ethandiol, Butandiol oder 2-Ethylhexandiol. Neben diesen Co-Katalysatoren lassen sich auch weitere basisch reagierende

Substanzen mitverwenden, wie Alkali- und Erdalkalihydroxide, Alkalialkoholate und -Phenolate, sowie Alkali- und Erdalkalisalze von Carbon- bzw. höheren Fettsäuren.

Selbstverständlich ist es auch möglich, Mischungen verschiedener Co-Katalysatoren einzusetzen oder andere Katalysatorsysteme mitzuverwenden, die in der Lage sind, die Isocyanatreaktionen zu beschleunigen, wie metallorganische Verbindungen von Zinn, Antimon oder Blei. Vorzugsweise werden jedoch solche verwendet, die bei der Trimerisation eingebaut werden, wie Carbamidsäurederivate bildende Hydroxyverbindungen oder solche, die ebenfalls, wie die verwandten Trimerisierungskatalysatoren, thermisch desaktivierbar sind, so z.B. die Mannichbasen.

Die erfindungsgemäße Umsetzung erfolgt bei 30 - 110°C vorzugsweise 40 - 90°C. Die Umsetzung wird im allgemeinen, von den geringen Lösungsmittelmengen für den Katalysator abgesehen, lösungsmittelfrei durchgeführt, obwohl selbstverständlich die Mitverwendung der an sich bekannten Lacklösungsmittel prinzipiell nicht ausgeschlossen ist.

Im allgemeinen wird die Katalysatorenmenge so bemessen, daß das erhaltene Reaktionsgemisch (ohne gegebenenfalls mitverwendete Lösungsmittel) einen NCO-Gehalt von ca. 20 bis 30 Gew.-% aufweist. Nicht umgesetztes, überschüssiges Ausgangsdiisocyanat kann anschließend in an sich bekannter Weise, beispielsweise durch Dünnschichtdestillation, entfernt werden.

Das erfindungsgemäße Verfahren kann beispielsweise wie folgt durchgeführt werden:

Das Ausgangsdiisocyanat wird in einem Dreihalskolben unter Stickstoff (die Mitverwendung von Inertgas ist nicht unbedingt erforderlich) vorgelegt und auf eine Temperatur im Bereich von 40 bis 70°C, beispielsweise 60°C, erwärmt. In diese Vorlage trägt man die Katalysatorlösung ein. Die Trimerisierung beginnt augenblicklich, nachdem die Katalysatorlösung im Ausgangsdiisocyanat eingerührt ist. Die Temperatur steigt langsam innerhalb von 30 bis 60 Minuten auf 70-80°C. Bei dieser Temperatur wird so lange nachgerührt, bis das Reaktionsgemisch den angestrebten NCO-Gehalt von ca. 20 bis 30 Gew.-% aufweist, wobei gegebenenfalls weitere Katalysatormengen portionsweise zugegeben werden, um den Anteil des sich thermisch zersetzenden Katalysators zu ersetzen. Die Reaktion kann durch Zugabe eines Abstoppers oder durch kurzzeitiges Erhitzen auf über 80°C liegende Temperaturen unterbrochen werden. Selbstverständlich kann man auch dergestalt verfahren, daß man das Ausgangsdiisocyanat auf eine über 80°C liegende Temperatur erhitzt und den Katalysator portionsweise bis zum Erreichen des gewünschten NCO-Gehalts des Reaktionsgemisches von ca. 20 bis 30 Gew.-% zugibt. Ebenso ist es möglich, die Reaktion kontinuierlich, etwa in kaskadenförmig angeordneten Reaktoren, durchzuführen.

Diese Art der Durchführung des erfindungsgemäßen Trimerisierungsverfahrens empfiehlt sich insbesondere bei Verwendung der besonders bevorzugten, thermisch labilen, Hydroxyalkyl-Substituenten aufweisen quaternären Ammoniumhydroxide. Selbstverständlich ist es jedoch auch denkbar, das erfindungsgemäße Verfahren unter Verwendung

von thermisch weitgehend stabilen Katalysatoren des Standes der Technik durchzuführen und die Trimerisierungsreaktion durch Zugabe eines geeigneten Katalysatorengifts abzustoppen.

Nach Beendigung der Trimerisierungsreaktion erfolgt vorzugsweise eine destillative Entfernung des noch im Gemisch vorliegenden Ausgangsdiisocyanates unter Verwendung eines Dünnschichtverdampfers, so daß als Destillationsrückstand die erfindungsgemäßen Isocyanato-isocyanurate mit einem Monomerengehalt von weniger als 3 Gew.-%, vorzugsweise von weniger als 0,7 Gew.-% anfallen.

In Abhängigkeit vom Trimerisierungsgrad, bzw. von der Molekulargewichtsverteilung fallen die erfindungsgemäßen Isocanato-isocyanurate in monomerenfreier Form als hochviskose bzw. bei Raumtemperatur feste Harze mit einem bei ca. 60-70°C liegenden Schmelzpunkt und einem NCO-Gehalt zwischen 9 und 15 Gew.-% an.

Die IR-Spektren der erfindungsgemäßen Isocyanato-isocyanurate beweisen deren Isocyanuratstruktur durch Absorptionen bei 1684 $cm^{-1}$ für die C = O - Schwingung und bei 1450 $cm^{-1}$ für die "Ringbande" des gebildeten Sechsringes. Uretdion-Einheiten können nicht nachgewiesen werden.

Nach gelchromatographischen Untersuchungen hängt der Gehalt an reinem Trimeren, bestehend aus drei Monomer-Einheiten, vom jeweiligen Trimierisierungsgrad ab, beträgt im allgemeinen jedoch über 30 %. Da es sich bei den

erfindungsgemäßen Isocyanato-isocyanurate um Isomeren- bzw. Homologengemische handelt, stellt "n" in der allgemeinen Formel für die erfindungsgemäßen Verbindungen eine ganze oder (im statistischen Mittel) gebrochene Zahl von 1 bis 5 dar. Wegen des genannten hohen Gehalts der erfindungsgemäßen Verbindungen an reinen Trimeren steht "n" vorzugsweise für eine ganze oder gebrochene Zahl zwischen 1 und 4.

Da davon ausgegangen werden kann, daß die beim erfindungsgemäßen Verfahren einzusetzenden Diisocyanate Isocyanatgruppen einer ungefähr gleichen Reaktivität aufweisen, muß angenommen werden, daß in den erfindungsgemäßen Isocyanato-isocyanuraten sowohl mit dem, der Struktur der eingesetzten Diisocyanate entsprechenden, monocyclischen Cyclopentanring als auch mit dem, der Struktur der eingesetzten Diisocyanate entsprechenden, bicyclischen Cycloheptan-Ringsystem verknüpfte Isocyanatomethyl-Gruppen vorliegen.

Die erfindungsgemäßen Isocyanato-isocyanurate zeichnen sich besonders durch geringe Eigenfärbung, hohe Funktionalität und sehr hohe Reaktivität aus. Sie sind bei Raumtemperatur fest und eignen sich in mit geeigneten Blockierungsmitteln blockierter Form als Härter für Pulverlacke. Ein mit $\varepsilon$-Caprolactam stöchiometrisch blockiertes Produkt (aus Beispiel 6) hat beispielsweise einen Schmelzbereich von 90 bis 125°C und bleibt als Pulver selbst bei 40°C Lagertemperatur über Wochen rieselfähig.

Die Produkte zeigen gute Löslichkeiten in für Lackanwendungen üblichen Lösungsmitteln (Ethylglykolacetat, Ethylglykolacetat/Xylol 1 : 1, Essigsäureethylester, -butylester) und können daher gut zur Herstellung von hochwertigen Beschichtungssystemen, z.B. Zweikomponenten-Polyurethanlacken eingesetzt werden, die sich durch ihre Witterungsbeständigkeit, vor allem aber durch ihre hohe UV-Beständigkeit auszeichnen.

Weiterhin weisen die erfindungsgemäßen Trimerisate eine gute Verträglichkeit, bzw. hohe Verdünnbarkeit mit unpolaren Lösungsmitteln auf, die sie für die Anwendung im Autoreparaturlacksektor hervorragend geeignet machen.

Bevorzugte Reaktionspartner für die erfindungsgemäßen, ggf. in blockierter Form vorliegenden Verfahrensprodukte bei der Herstellung von Polyurethanlacken sind die in der Polyurethantechnik an sich bekannten Polyhydroxypolyester (wobei insbesondere Alkydharze in Frage kommen), Polyhydroxypolyacrylate, und gegebenenfalls niedermolekularen, mehrwertigen Alkohole. Auch Polyamine, insbesondere in blockierter Form als Polyketimine oder Oxazolidine, sind denkbare Reaktionspartner für die erfindungsgemäßen Verfahrensprodukte. Die Mengenverhältnisse, in denen die erfindungsgemäßen, ggf. blockierten Polyisocyanate und die genannten Reaktionspartner bei der Herstellung von Polyurethanlacken umgesetzt werden, werden im allgemeinen so gewählt, daß auf eine (ggf. blockierte) Isocyanatgruppe 0,8-3, vorzugsweise 0,9-1,1, Hydroxyl-, Amino-, und/oder Carboxylatgruppen entfallen.

Zur Beschleunigung der Aushärtung können in bekannter Weise die in der Isocyanatchemie üblichen Katalysatoren verwendet werden, wie z.B. tert. Amine wie Triethylamin, Pyridin, Methylpyridin, Benzyldimethylamin, N,N-Dimethylaminocyclohexan, N-Methylpiperidin, Pentamethyldiethylentriamin, N,N'-Endoethylpiperazin, N,N'-Dimethylpiperazin usw. Metalisalze wie Eisen (III)-chlorid, Zinkchlorid, Zink-2-ethylcaproat, Zinn(II)-2-ethylcaproat, Dibutylzinn(IV)-dilaurat, Molybdänglykolat usw.

Bei Verwendung der erfindungsgemäßen Verfahrensprodukte in Einbrennlacken werden die NCO-Gruppen ganz oder teilweise in bekannter Weise blockiert. Das Polyisocyanat wird mit einem geeigneten Blockierungsmittel, vorzugsweise bei erhöhter Temperatur (z.B. 40 bis 140°C) ggf. in Gegenwart eines geeigneten Katalysators, wie z.B. tert. Amine, Metallsalze wie Zink-2-ethylcaproat, Zinn(II)-2-ethylcaproat, Dibutylzinn(IV)-dilaurat oder Alkaliphenolat umgesetzt.

Geeignete Blockierungsmittel sind beispielsweise: Monophenole wie Phenol, die Kresole, die Trimethylphenole, die tert. Butylphenole; tert. Alkohole wie tert. Butanol, tert. Amylalkohol, Dimethylphenylcarbinol; leicht Enole bildende Verbindungen wie Acetessigester, Acetylaceton, Malonsäurederivate wie Malonsäurediester mit 1 bis 8 C-Atomen in den Alkoholresten; sekundäre aromatische Amine wie N-Methylanilin, die N-Methyltoluidine, N-Phenyltoluidin, N-Phenylxylidin; Imide wie Succinimid; Lactame wie ε-Caprolactam, δ-Valerolactam; Oxime wie

Butanonoxim, Cyclohexanonoxim; Mercaptane wie Methylmercaptan, Ethylmercaptan, Butylmercaptan, 2-Mercaptobenzthiazol, $\alpha$-Naphthylmercaptan, Dodecylmercaptan, Triazole wie IH-1,2,4-Triazol.

Zur Herstellung der Lackbindemittel werden ggf. blockiertes Polyisocyanat, polyfunktioneller Reaktionspartner, Katalysator und ggf. die üblichen Zusätze wie z.B. Pigmente, Farbstoffe, Füllstoffe und Verlaufmittel miteinander auf einem üblichen Mischaggregat, wie z.B. auf einer Sandmühle entweder mit oder ohne Lösungs- und Verdünnungsmittel gut vermischt und homogenisiert.

Die Anstrich- und Überzugsmittel können in Lösung oder aus der Schmelze, oder in fester Form nach den üblichen Methoden wie z.B. Streichen, Rollen, Gießen, Spritzen, dem Wirbelsinterverfahren oder dem elektrostatischen Pulversprühverfahren auf den zu beschichtenden Gegenstand aufgebracht werden.

Die die erfindungsgemäßen Polyisocyanate enthaltenden Lacke ergeben Filme, die überraschend gut auf metallischem Untergrund haften, besonders lichtecht, wärmefarbstabil und sehr abriebfest und, sofern sie in lufttrocknenden Lacken verwendet werden, besonders rasch, selbst bei Temperaturen um 0°C antrocknen. Darüberhinaus zeichnen sie sich durch große Härte, Elastizität, sehr gute Chemikalienbeständigkeit, hohen Glanz, ausgezeichnete Wetterbeständigkeit und gute Pigmentierbarkeit aus.

Die folgenden Beispiele erläutern die Erfindung. Alle Prozentangaben betreffen Gewichtsprozente. Als Ausgangsmaterial wurde jeweils ein gemäß Beispiel 2 der DE-OS 1 645 595 hergestelltes 3(4), 8(9)-Bis(isocyanatomethyl)-tricyclo [5,2,1,$0^{2,6}$]-decan (Tricyclodecyldiisocyanat) (TCDI) verwendet. In den Beispielen werden außerdem die Katalysatorlösungen A, B und C eingesetzt:

A: N,N,N-Trimethyl-N(2-hydroxyethyl)-ammoniumhydroxid, hergestellt durch Umsetzung von Trimethylamin und Ethylenoxid in wässrig-methanolischem Medium, ca. 5 % gelöst in einem Lösungsmittelgemisch aus 8 Teilen Dimethylformamid und 1 Teil Methanol.

B: Wie A, jedoch gelöst in 2-Ethylhexanol/Methanol (4:1).

C: N-(2-hydroxyethyl)-N,N-dimethyl-N(2,2 -dihydroxymethylbutyl)-ammoniumhydroxid, hergestellt durch Umsetzung des entsprechenden Amins mit Ethylenoxid in wässrig-methanolischem Medium, ca. 10 % in 2-Ethylhexanol/Methanol (8 : 1) gelöst.

Beispiel 1:

246 g ( 1 Mol) TCDI wird bei 60°C mit 5 ml Katalysatorlösung A versetzt. Die Reaktion verläuft sofort exotherm, die Temperatur des Reaktionsgemisches steigt bis auf 67°C, um dann allmählich wieder zu sinken. Nach einer Stunde beträgt die Temperatur 60°C, der NCO-Gehalt ist auf 31,0 % gesunken. Nach erneuter Zugabe von 5 ml Katalysatorlösung erreicht der Ansatz bei ähnlichem Reaktionsverlauf nach insgesamt 3,5 h einen NCO-Gehalt von 27,3 %. Die Reaktion wird durch Zugabe von 1 ml einer Lösung aus 1 ml Perfluorbutansulfonsäure in 2 ml Dimethylformamid unterbrochen.

Nach Dünnschichtdestillation erhält man ein Harz geringer Eigenfärbung, das als 75 % Lösung in Ethylglykolacetat/Xylol (1 : 1) einen NCO-Gehalt von 7,3 % hat.

Beispiel 2:

Wie in Beispiel 1 beschrieben, werden 1053 g TCDI mit dreimal 10 ml und einmal 5 ml Katalysatorlösung A versetzt. Bei einer Reaktionstemperatur von 60 bis 70°C erreicht das Gemisch in 8 Stunden einen NCO-Gehalt von 27,3 %. Durch Zugabe von 1 ml Perfluorbutansulfonsäure, gelöst in 2 ml Dimethylformamid, wird die Reaktion gestoppt und das Produkt durch Dünnschichtdestillation von Monomeren befreit. Das Harz hat als nahezu farblose 70 % Lösung in Ethylglykolacetat/Xylol (1 : 1) einen NCO-Gehalt von 7,1 % und eine Viskosität von 110 mPas (bei 21°C).

Beispiel 3:

492 g (2 Mol) TCDI werden wie in Beispiel 1 beschrieben, bei 60 bis 70°C mit insgesamt 30 ml Katalysatorlösung C umgesetzt. Nach 6 Stunden wird die Reaktion durch Zugabe eines Abstoppers (1 ml Perfluorbutansulfonsäure in 2 ml Dimethylformamid) bei einem NCO-Gehalt des Gemisches von 27,6 % unterbrochen. Nach Dünnschichtdestillation und Lösen des Harzes in Ethylglykolacetat/Xylol 1 : 1 (80 %) erhält man eine schwach gelb gefärbte Lösung mit 9,7 % NCO.

Beispiel 4:

492 g (2 Mol) TCDI werden bei 75°C mit 5 ml Katalysatorlösung A versetzt. Die Temperatur des Reaktionsgemisches steigt auf 79°C an und wird nach Abklingen der exothermen Reaktion durch äußere Heizung noch eine Stunde auf 80°C gehalten. In der gleichen Weise wird noch zweimal mit je 5 ml Katalysatorlösung katalysiert. Nach dem letzten Nachrühren wird zur Zerstörung des restlichen Katalysators das Reaktionsgemisch eine Stunde auf 90°C erhitzt. Der NCO-Wert des Gemisches beträgt dann 27,9 % und ändert sich auch bei weiterem Nachrühren nicht mehr.

Beispiel 5:

7380 g (30 Mol) TCDI werden bei 62°C mit 100 ml Katalysatorlösung B versetzt. Die Reaktion verläuft sofort exotherm und wird bei Erreichen von 76°C durch Kühlung

auf 70°C gebracht. Durch weitere, gelegentliche Kühlung wird die Reaktionstemperatur bei 70-75°C gehalten. Im Verlauf von 3,3 Stunden sinkt der NCO-Gehalt der Lösung auf 26,3 %. Die Reaktion wird durch Zugabe von 8 ml Perfluorbutansäure in 10 ml Dimethylformamid unterbrochen. Der NCO-Gehalt beträgt dann 25,7 % . Das durch Dünnschichtdestillation von Monomeren weitgehend befreite Produkt (Restmonomerengehalt: 0,53 %) besitzt als 75 % Lösung in Ethylglykolacetat / Xylol (1 : 1) einen NCO-Gehalt von 10,3 % und eine Viskosität von 4630 mPas (23°C).

Mit der genannten Lösung des Trimerisates gemäß Beispiel 5 werden unter Verwendung von in der Lacktechnologie üblichen, Hydroxylgruppen aufweisenden Polyacrylat- bzw. Polyesterharzen gebrauchsfertige Lacke mit jeweils 50 Gew.-% Bindemittelanteil hergestellt und deren lackechnischen Eigenschaften ermittelt. Zum Vergleich wurden entsprechende Lacke unter Verwendung von Isocyanatoisocyanuraten gemäß Stand der Technik geprüft. Bei diesen Versuchen wurden folgende Ausgangsmaterialien eingesetzt:

Polyacrylatharz: Copolymerisat aus Styrol, Butylacrylat und Hydroxyethylmethacrylat mit einem Hydroxylgehalt von 3,7 Gew.-% (80 % Lösung in Ethylglykolacetat) und einem mitteleren osmometrisch bestimmten Molekulargewicht von 2500.

Polyesterharz: Kondensationsprodukt aus Phthalsäure, Isophthalsäure, Hexandiol und Trimethylolpropan mit einem Gehalt an Hydroxylgruppen von 3,5 Gew.-% (70 % in Ethylglykolacetat / Xylol 1 : 1) und einem osmometrisch bestimmten mittleren Molekulargewicht von 1150.

Polyisocyanat I: Trimerisat von Hexamethylendiisocyanat mit einem Restmonomerengehalt von unter 0,7 Gew.-% und einem NCO-Gehalt von 20,0 Gew.-% (als 90 % Lösung in Ethylglykolacetat).

Polyisocyanat II: Trimerisat von Isophorondiisocyanat mit einem Restmonomerengehalt von unter 0,7 Gew.-% und einem NCO-Gehalt von 11,5 Gew.-% (als 70 % Lösung in Ethylglykolacetat/Xylol 1 : 1).

30,4 Gew.-Teile der Trimerisat-Lösung gemäß Beispiel 5 werden mit 34,0 Gew.-Teilen der Polyacrylatharz-Lösung (NCO/OH-Äquivalentverhältnis = 1 : 1) in 32,6 Gew.-Teilen eines Lösungsmittelsgemisches aus Toluol, Butylacetat, Ethylacetat und Ethylglykolacetat (Gew.-Verhältnis: 1 : 1 : 1 : 1), gelöst. Zu dieser Lösung werden 1 Teil eines Verlaufsmittels auf Basis Silikonöl (10 % in Ethylglykolacetat) und 2 Teile Katalysator (Zinkoctoat, 10 % Lösung in Ethylglykolacetat) hinzugefügt.

In einem Parallelversuch wird eine entsprechende Lacklösung unter Verwendung von 18,9 Gew.-Teilen der Polyisocyanat-Lösung I (NCO/OH-Äquivalentverhältnis = 1 : 1) hergestellt.

Mit diesen gebrauchsfertigen Lacken werden Glasplatten beschichtet (Schichtdicke nach Trocknung: 40-50 µm). Die jeweils gemessenen anwendungstechnischen Daten werden in nachstehender Tabelle I aufgeführt.

Tabelle I

| Abmischung / Film aus | erfindungsgemäßem Trimerisat | Trimerisat aus Hexamethylendiisocyanat |
|---|---|---|
| Standzeit der Lacke* (h) | 12,0 | 3,8 |
| physik. Trocknung (h) DIN 53 150 | 2 | 6 |
| chem. Trocknung (h) DIN 53 150 | 8 | 10 |
| Pendelhärte (nach König)(sec.) DIN 53 157 | | |
| 30´/80°C | 170 | 165 |
| 30´/120°C | 186 | 185 |
| 30´/120°C+ 16 h / 60°C | 205 | 188 |

* in direktem Vergleichsversuch wurden die Gelierzeiten ermittelt, d.h. die Zeiten, bis die Abmischungen eine Viskosität von $\eta^{23}$ = 20000 mPas erreichten.

In einer zweiten Versuchsserie wurden entsprechende gebrauchsfertige Lacke unter Verwendung von jeweils 37,6 Gew.-Teilen der Polyersterharz-Lösung und 31,6 Gew.-% der Lösung des erfindungsgemäßen Trimerisates aus

Beispiel 5 bzw. unter Verwendung von 40,7 Gew.-% Polyesterlösung und 20,7 Gew.-% des Polyisocyanates II (jeweiliges NCO/OH-Äquivalentverhältnis = 1 : 1) bei ansonsten gleicher Rezeptur hergestellt. Es wurden folgende lacktechnischen Eigenschaften ermittelt:

1. Elastizität

Elastizitätsbestimmungen beider Filme nach DIN 53 156 ergaben für den Lack auf Basis des bekannten Trimerisats einen Wert von

6,9 mm,

für den auf Basis des erfindungsgemäßen Trimerisats

9,5 mm.

2. Schlagfestigkeit

Die Bestimmung der Schlagfestigkeit nach ASTM D 779 - 69 ergab

10 inch pound

für das bekannte und

80 inch pound

für das neue Produkt.

Mit Hilfe der neuen Produkte ist es damit möglich, widerstandsfähige Beschichtungen mit sehr guten mechanischen Eigenschaften herzustellen, die zudem noch eine sehr gute Elastizität aufweisen, also nicht leicht zu Versprödungen neigen.

3) Trocknung (Bestimmung nach DIN 53 150)
Bei sonst vergleichbaren Eigenschaften beider Produkte, bzw. Filme (gute bis sehr gute Lösungsmittelbeständigkeit gegen übliche Lösungsmittel wie Toluol, Essigsäureethylester, Ethylglykolacetat, Aceton) zeigt sich auch hier überraschenderweise ein sehr großer Vorteil der erfindungsgemäßen Trimerisate:
Bei sehr guter physikalischer Antrocknung beider Filme ($T_1$) zeigten Filme unter Verwendung der erfindungsgemäßen Trimerisate eine deutlich beschleunigte chemische Trocknung, d.h. Aushärtung durch chemische Reaktion ($T_2$), als Filme unter Verwendung von Isophoron-diisocyanat-Trimerisat.

| | $T_1$ (h) | $T_2$ (h) |
|---|---|---|
| Film aus bekanntem Trimerisat | 1 | 6 |
| Film aus erfindungsgemäßem Trimerisat | 1 | 4,5 |

Somit ist es durch Verwendung der erfindungsgemäßen Harze möglich, Beschichtungen herzustellen, die innerhalb kürzester Zeit voll belastbar, bzw. überarbeitbar sind. Neben verringerten Standzeiten, während der frisch beschichtete Produkte vor äußeren Einflüssen geschützt werden müssen, und damit erhöhtem Durchsatz, bzw. geringerem Lageraufwand, ergeben sich damit offensichtliche Vorteile vor allem bei der Lackierung großer, sperriger Gebrauchsgüter, die nicht bei erhöhter Temperatur ausgehärtet werden können.

Beispiel 6

919 g TCDI werden bei 60°C mit 13 ml Katalysatorlösung B versetzt. Nach Abklingen der sofort einsetzenden exothermen Reaktion wird das Gemisch auf 75°C erwärmt. Nach einer Stunde beträgt der NCO-Gehalt 30,7 %. Im Verlaufe von weiteren sechs Stunden werden bei 75°C noch insgesamt 18 ml Katalysatorlösung in drei Portionen zugegeben. Danach wird die Reaktion durch Zugabe von 1 ml Perfluorbutansulfonsäure, gelöst in 1 ml Dimethylformamid, abgestoppt. Durch Dünnschichtdestillation wird das Rohprodukt von Monomeren weitgehend befreit (Restmonomerengehalt: 0,48 %). Man erhält ein festes, wenig gefärbtes Harz (Schmelzbereich: 60-68°C), das einen NCO-Gehalt von 12,9 % aufweist.

300 g des so erhaltenen Produkts werden mit 104 g ε-Caporolactam in bekannter Weise verkappt. Man erhält einen spröden Feststoff (Schmelzbereich: 90 - 125°C), der gut zerkleinert werden kann und auch bei Lagerung bei 40°C über mindestens drei Wochen rieselfähig bleibt. Der Gehalt an verkappten NCO-Gruppen beträgt 9,6 %.

Patentansprüche:

1. Isocyanato-isocyanurate der Formel

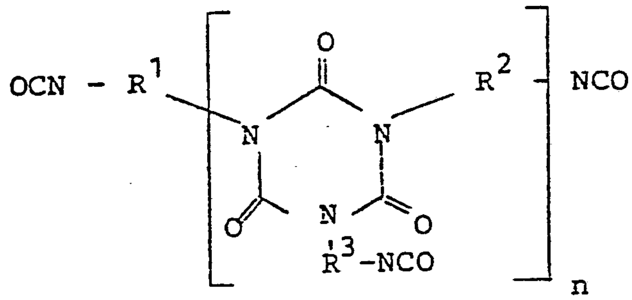

in welcher

$R^1$, $R^2$, und $R^3$ für gleich oder verschiedene Reste stehen und isomere Kohlenwasserstoffreste der Formeln

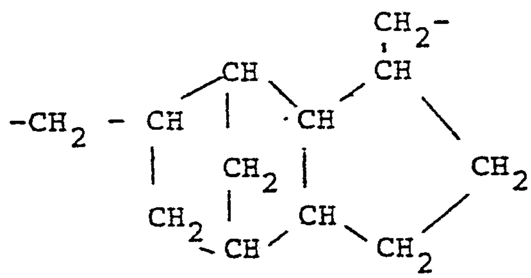

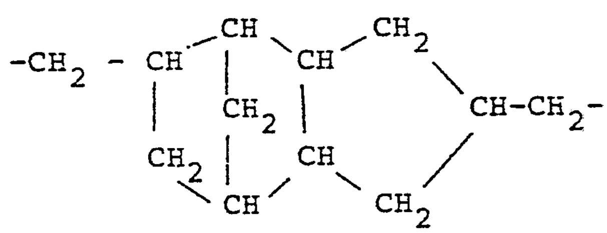

oder

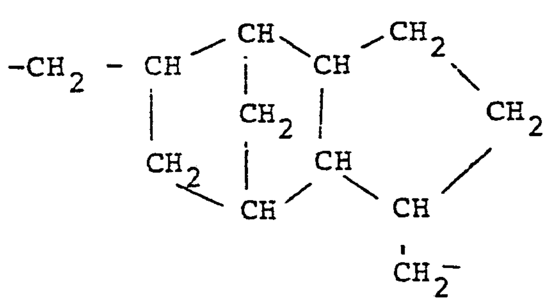

bedeuten

und

n für eine ganze oder (im statistischen Mittel) gebrochene Zahl von 1 bis 5 steht.

2. Verfahren zur Herstellung von Isocyanato-isocyanuraten gemäß Anspruch 1, dadurch gekennzeichnet, daß man einen Teil der Isocyanatgruppen von 3 (4), 8 (9)-Bis(-isocyanatomethyl)-tricyclo [5, 2, 1, $0^{2,6}$]-decan in Gegenwart von die Trimerisierung von Isocyanatgruppen beschleunigenden Katalysatoren trimerisiert.

3. Verfahren gemäß Anspruch 2, dadurch gekennzeichnet, daß man als die Trimerisierung von Isocyanatgruppen beschleunigende Katalysatoren quartäre Ammoniumhydroxide verwendet.

4. Verfahren gemäß Ansprüchen 2 und 3, dadurch gekennzeichnet, daß man als die Trimerisierung von Isocyanatgruppen beschleunigende Katalysatoren quartäre Ammoniumhydroxide einsetzt, welche mindestens ein mit mindestens einer Hydroxyalkylgruppe substituiertes quaternäres Stickstoffatom aufweisen.

5. Verwendung der Isocyanato-isocyanurate gemäß Anspruch 1, gegebenenfalls in mit Blockierungsmitteln für Isocyanatgruppen blockierter Form, als Isocyanatkomponente in Polyurethanlacken.

Europäisches Patentamt

**0039864**
Nummer der Anmeldung

## EUROPÄISCHER RECHERCHENBERICHT

EP 81103339.8

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - A1 - 2 737 211 (BAYER)<br>* Patentanspruch 1 *<br>-- | 1 | C 07 D 251/34<br>C 09 D 3/72<br>C 08 G 18/75 |
| | DE - A - 2 318 170 (BAYER)<br>* Patentanspruch 1*<br>-- | 1 | |
| | DE - A - 1 816 521 (MARATHON)<br>* Zusammenfassung *<br>-- | 1 | |
| | DE - B - 1 695 543 (MARATHON)<br>* Patentanspruch *<br>-- | 1 | RECHERCHIERTE SACHGEBIETE (Int. Cl.³) |
| | DE - B - 1 695 520 (BAYCHEM)<br>* Patentansprüche *<br>-- | 1,5 | C 07 D 251/00<br>C 09 D 3/00<br>C 08 G 18/00 |
| | DE - B - 1 146 889 (JCJ)<br>* Spalte 1; Spalte 2, Abs. 1 *<br>-- | 1 | |
| | DE - A - 2 241 299 (MARATHON)<br>* Patentanspruch 1; Fig. 1 *<br>---- | 5 | |

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 04-08-1981 | HAMMER |